# EUROPEAN PATENT SPECIFICATION

(11) **EP 4 134 001 B1**
(45) Date of publication and mention of the grant of the patent: **01.10.2025**
(21) Application number: 21796316.4
(22) Date of filing: 25.04.2021
(51) Int. Cl.: A61B 5/024, A61B 5/1455

(54) **OPTICAL EMITTER AND OPTICAL SENSING DEVICE**
OPTISCHER EMITTER UND OPTISCHE MESSVORRICHTUNG
ÉMETTEUR OPTIQUE ET DISPOSITIF DE DÉTECTION OPTIQUE

(30) Priority: 29.04.2020 CN 202010358579
(43) Date of publication of application: 15.02.2023
(73) Proprietor: Huawei Technologies Co., Ltd., Shenzhen, Guangdong 518129 (CN)
(72) Inventor: YANG, Sulin, Shenzhen, Guangdong 518129 (CN); CHENG, Yuanbing, Shenzhen, Guangdong 518129 (CN)
(74) Representative: Maiwald GmbH
(86) International application number: PCT/CN2021/089645
(87) International publication number: WO 2021/218861

(56) References cited:
- CN-A- 102 802 509
- CN-A- 106 308 776
- CN-A- 107 480 590
- CN-A- 110 598 656
- KR-A- 20060 012 844
- US-A1- 2016 238 439
- US-A1- 2017 164 848
- US-A1- 2017 372 152
- US-A1- 2019 269 341
- US-A1- 2019 365 231

## Description

### TECHNICAL FIELD

This application relates to the field of detection technologies, and in particular, to an optical transmitter and a photosensitive apparatus.

### BACKGROUND

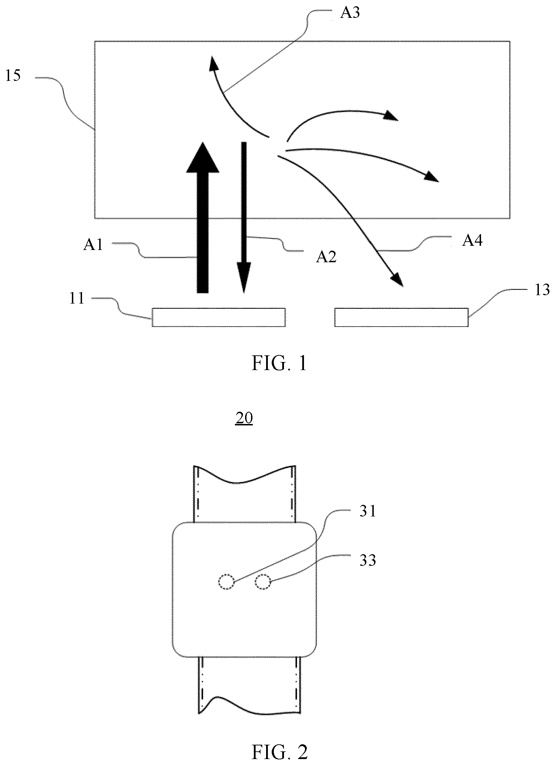

Photoplethysmography (photoplethysmograph, PPG) is an optical measurement technology, and is widely used to measure or monitor a vital sign of a human body, for example, a heart rate (HR), a respiratory rate (RR), or oxygen saturation. FIG. 1 shows an existing photosensitive apparatus for which a PPG technology is used and in which a light source 11 and a photodetector 13 are disposed at an interval. The light source 11 is configured to transmit a test optical signal (A1 shown in FIG. 1) to a skin 15. Most of light is reflected inside the skin or in a skin interface (A2 shown in FIG. 1), and returns to the light source 11. A small part of light (A3 shown in FIG. 1) is reflected and/or scattered inside the skin. A part of the reflected and/or scattered light (A4 shown in FIG. 1) returns to the photosensitive apparatus and is received by the photodetector 13. This part of the reflected and/or scattered light is referred to as a backward reflected and/or scattered signal. There is maximum light intensity at a center of a light spot output by the light source 11, and light intensity becomes weaker as a shifting distance from the center increases. Similarly, strength of the backward reflected and/or scattered signal also decreases as the shifting distance increases. In other words, a strength distribution curve of the test optical signal output by the light source 11 and a strength distribution curve of the backward reflected and/or scattered signal each are approximately in a single-bell shape. Because the photodetector 13 needs to be separated from the light source 11 by a specific distance, most test optical signals transmitted by the light source 11, especially an optical signal at a center at which light intensity of a test optical signal is high and an optical signal in an adjacent region of the center, are reflected to the light source 11 to be consumed, and cannot be used. Consequently, the photodetector 13 can receive only a small quantity of effective backward reflected and/or scattered signals. In this case, utilization of the light source 11 is low, and power consumption of the photosensitive apparatus is high. The US 2017 / 016 48 48 A1 refers to a light field management for an optical biological parameter

### SUMMARY

The present invention is directed to an optical transmitter used for photoplethysmography as defined in the independent claim. Further aspects of the invention are defined in the dependent claims.

Embodiments of this application provide an optical transmitter and a photosensitive apparatus, to improve utilization of a light source.

According to a first aspect of this application, an optical transmitter used for photoplethysmography is provided, including a light source and an adjustment structure. The light source is configured to output an original light spot, to transmit a test optical signal to a skin of a user, the adjustment structure is located on an output optical path of the test optical signal, a test optical signal transmitted from an original light spot center of the original light spot is a central light spot optical signal, and the adjustment structure is configured to scatter the central light spot optical signal in a direction away from the original light spot center, to convert the original light spot into a test light spot.

In the first aspect of this application, the adjustment structure is used to scatter the central light spot optical signal of the original light spot in the direction away from the original light spot center, to adjust a light intensity distribution of the light spot, enhance light intensity in a region outside a center of the test light spot, and improve strength of a backward signal. In other words, an original test optical signal beam transmitted by the optical transmitter is adjusted, to improve utilization of the central light spot optical signal of the original light spot, so that a photodetector can receive more effective backward signals, to reduce a loss of the optical transmitter.

According to the first aspect, in a first possible implementation of the first aspect, a light exit structure disposed on the light source is further included, and the central light spot optical signal is incident to the adjustment structure by using the light exit structure. In other words, the adjustment structure is disposed on an original output optical path of the central light spot optical signal, to change the output optical path of the central light spot optical signal. In this case, a structure of the optical transmitter is simplified.

According to the first aspect or the first to the second possible implementations of the first aspect, in a third possible implementation of the first aspect, the adjustment structure includes a grating structure, configured to diffract the incident central light spot optical signal; and/or the adjustment structure includes a reflective film, configured to reflect the incident central light spot optical signal. The original light spot can be converted into the test light spot by using a simple grating structure and/or reflective film, so that the optical transmitter maintains a simple structure when utilization of the light source is improved, to help reduce occupied space of the optical transmitter, and help miniaturize the optical transmitter.

According to the first aspect or the first to the third possible implementations of the first aspect, in a fourth possible implementation of the first aspect, the adjustment structure includes a lens structure, the lens structure includes an incident side and an emergent side, the incident side is disposed toward the light source, a recess that is concavely disposed toward a side on which the light source is located is disposed on the emergent side of the lens structure, and the central light spot optical signal is incident to the lens structure from an orthogonal projection coverage of the recess on the incident side, so that the central light spot optical signal can be effectively scattered or shifted in a direction away from the original light spot center, to improve utilization of the central light spot optical signal.

For example, the recess is a spherical surface. The central light spot optical signal is incident to the adjustment structure, and then is scattered in the direction away from the original light spot center under a scattering action of the spherical surface. For example, the recess includes a slope, and the central light spot optical signal is transmitted from another region other than the recess on the emergent side under a total internal reflection action of the slope.

According to the first aspect or the first to the fourth possible implementations of the first aspect, in a fifth possible implementation of the first aspect, the adjustment structure includes a lens structure, the lens structure includes an incident side and an emergent side, the incident side is disposed toward the light source, a recess that is concavely disposed toward a side away from the light source is disposed on the incident side of the lens structure, and the central light spot optical signal can be incident to the lens structure from the recess and be transmitted from the emergent side, so that the central light spot optical signal can be effectively scattered in a direction away from the original light spot center, to improve utilization of the central light spot optical signal.

According to the first aspect or the first to the fifth possible implementations of the first aspect, in a sixth possible implementation of the first aspect, the incident side is fastened to the light exit structure, and the lens structure directly grows on the light source, to help reduce a volume of the optical transmitter. The test optical signal transmitted by the light source directly enters the lens structure, to reduce a loss of the test optical signal in a transmission process.

According to the first aspect or the first to the sixth possible implementations of the first aspect, in a seventh possible implementation of the first aspect, the emergent side includes a spherical surface protruding in a direction away from the optical transmitter. Under a convergence action of the spherical surface, a scattering angle of the overall test optical signal is reduced.

According to the first aspect or the first to the seventh possible implementations of the first aspect, in an eighth possible implementation of the first aspect, the adjustment structure includes a light exit structure disposed on the light source, and the light exit structure is disposed by avoiding an original output optical path of the central light spot optical signal, to adjust a light intensity distribution of the light spot. The light spot can be adjusted by using a simple optical path design, to help manufacture the optical transmitter.

According to the first aspect or the first to the eighth possible implementations of the first aspect, in a ninth possible implementation of the first aspect, there are at least two light exit structures, and two adjacent light exit structures are disposed at an interval, so that the test light spot is an N-point light spot, where N is greater than 1. The original light spot can be converted into an N-point light spot by simply setting a quantity of light exit structures, to further improve convenience of manufacturing the optical transmitter.

According to the first aspect or the first to the ninth possible implementations of the first aspect, in a tenth possible implementation of the first aspect, the light exit structure is an annular structure, and the test light spot is an annular light spot. The original light spot can be converted into an annular light spot by simply setting a shape of the light exit structure, to further improve convenience of manufacturing the optical transmitter.

According to the first aspect or the first to the tenth possible implementations of the first aspect, in an eleventh possible implementation of the first aspect, the adjustment structure includes a light exit window, the light exit window includes a first connection surface, an included angle between a normal line of the first connection surface and a first direction is a first included angle, the first included angle is an acute angle, the central light spot optical signal is incident from the first connection surface to the light exit window, and the first direction is perpendicular to an original direction in which the central light spot optical signal is transmitted from the original light spot. The first connection surface changes a refraction direction of the test optical signal in the light exit window, so that a direction inclines toward a direction of the photodetector after the test optical signal is transmitted from the light exit window, to allow more effective backward signals to enter the photodetector.

According to the first aspect or the first to the eleventh possible implementations of the first aspect, in a twelfth possible implementation of the first aspect, the light exit window further includes a second connection surface disposed by being connected to the first connection surface, the first connection surface is disposed close to the photodetector, an included angle between a normal line of the second connection surface and the first direction is a second included angle, and the second included angle is greater than the first included angle.

The original light spot may be considered as a centrosymmetric light spot. The original light spot center is used as a reference. A transmission direction of an optical signal on a side that is of the original light spot and that is close to the photodetector deviates toward the photodetector, and a transmission direction of an optical signal on a side that is of the original light spot and that is away from the photodetector deviates away from the photodetector. The second included angle is greater than the first included angle, so that an adjustment degree of a test optical signal incident to the first connection surface is greater than an adjustment degree of a test optical signal incident to the second connection surface, to lead more optical signals to the photodetector, and further allow an effective backward signal to enter the photodetector.

According to the first aspect or the first to the twelfth possible implementations of the first aspect, in a thirteenth possible implementation of the first aspect, the light exit window further includes a third connection surface, the third connection surface is connected to one end that is of the first connection surface and that is away from the second connection surface, an included angle between a normal line of the third connection surface and the first direction is a third included angle, the second included angle is an acute angle, and the third included angle is an obtuse angle. The third connection surface can be used to reduce a scattering angle of a test optical signal transmitted from a part that is of the original light spot and that is close to the photodetector. In other words, edge optical signals on a side that is of the original light spot and that is close to the photodetector converge, to reduce a loss of the light source.

According to the first aspect or the first to the thirteenth possible implementations of the first aspect, in a fourteenth possible implementation of the first aspect, a light intensity distribution curve of the original light spot is a single-bell curve, there is maximum light intensity at the original light spot center, a light intensity distribution curve of the test light spot is a double-bell curve, and light intensity at a center of the test light spot is less than maximum light intensity of the test light spot, to further improve utilization of the light source of the optical transmitter.

According to the first aspect or the first to the fifteenth possible implementations of the first aspect, in a sixteenth possible implementation of the first aspect, the recess may be a spherical surface. Under a scattering action of the spherical surface, the central light spot optical signal can be effectively scattered in a direction away from the original light spot center, to improve utilization of the central light spot optical signal.

According to the first aspect or the first to the sixteenth possible implementations of the first aspect, in a seventeenth possible implementation of the first aspect, a longitudinal section of the recess is in a cone shape, to change a transmission direction of the central light spot optical signal of the original light spot, and convert the original light spot into a test light spot.

According to a second aspect, this application provides a photosensitive apparatus, including the optical transmitter and the photodetector. The photodetector is configured to receive a backward signal obtained after a test optical signal is reflected and/or scattered by a skin of a user.

An original light spot is adjusted to a test light spot by using an adjustment structure, so that a central light spot optical signal of the original light spot is shifted in a direction away from an original light spot center, to lead more test optical signals to a direction in which the photodetector is located, allow the photodetector to receive more effective backward signals, and improve detection accuracy of the photosensitive apparatus. Because utilization of the central light spot optical signal is improved, power consumption of the photosensitive apparatus is reduced.

According to the second aspect, in a first possible implementation of the second aspect, the photodetector includes a detector body and an optical receiving window, the backward signal enters the detector body through the optical receiving window, the optical receiving window includes a first surface and a second surface, the second surface is disposed toward the photodetector, and the second surface includes an inclined surface inclining toward a direction in which the optical transmitter is located, to reduce an incident angle at which a backward signal transmitted from the second surface is incident to the detector body, so that more effective backward signals enter the detector body, to further improve detection accuracy of the photosensitive apparatus.

According to the second aspect or the first possible implementation of the second aspect, in a second possible implementation of the second aspect, the photodetector includes a detector body and an optical receiving window, the backward signal enters the detector body through the optical receiving window, the detector body and the optical receiving window each are of an annular structure, and the detector body and the optical receiving window each are disposed around the optical transmitter. All backward signals at a specific distance from the center within an annular range can be received by the photodetector, to improve utilization of a light source, improve performance of the photosensitive apparatus, and reduce power consumption of the photosensitive apparatus.

### BRIEF DESCRIPTION OF DRAWINGS

FIG. 1 is a schematic diagram of an application principle of an existing photosensitive apparatus;
FIG. 2 shows a device in which an example in content disclosed in this application may be implemented;
FIG. 3 is a block diagram of a structure of a photosensitive apparatus;
FIG. 4 is a schematic diagram of a partial structure of a photosensitive apparatus according to Implementation 1 of this application;
FIG. 5a is a schematic diagram of an original light spot output by a light source;
FIG. 5b is a schematic diagram of a light intensity curve distribution of the original light spot shown in FIG. 5a;
FIG. 6 is a schematic diagram of a structure of an optical transmitter of the photosensitive apparatus shown in FIG. 4;
FIG. 7a is a schematic diagram in which a test light spot is an annular light spot;
FIG. 7b is a schematic diagram of a light intensity curve distribution of the original light spot shown in FIG. 7a;
FIG. 7c is a schematic diagram in which a test light spot is a double-point light spot;
FIG. 7d is a schematic diagram in which a test light spot is a four-point light spot;
FIG. 7e is a top view in which two light exit structures are disposed at an interval in a light source;
FIG. 7f is a side view of the light source shown in FIG. 7e;
FIG. 7g is a top view in which a light exit structure of a light source is set to be annular;
FIG. 8a to FIG. 8h are schematic diagrams in which a partial structure of a photosensitive apparatus is used in contact with a skin of a user according to Implementation 2 of this application;
FIG. 9a to FIG. 9d are schematic diagrams in which a structure of a photosensitive apparatus is used in contact with a skin of a user according to Implementation 3 of this application;
FIG. 10a is a schematic diagram in which a structure of a photosensitive apparatus is used in contact with a skin of a user according to Implementation 4 of this application;
FIG. 10b to FIG. 10e are schematic diagrams of possible structures of an optical receiving window; and
FIG. 11 is a schematic diagram of a structure of a photosensitive apparatus according to Implementation 5 of this application.

### DESCRIPTION OF EMBODIMENTS

FIG. 2 shows a device in which an example in content disclosed in this application may be implemented. A photosensitive apparatus 20 shown in FIG. 2 is a wearable device that may be worn on a wrist of a user. A PPG technology is used for the photosensitive apparatus 20, and the photosensitive apparatus 20 is configured to measure or collect a vital sign of the user. The vital sign includes a heart rate, a respiratory rate, oxygen saturation, or the like. It can be understood that the photosensitive apparatus 20 is not limited to a wearable device, but may alternatively be another device that may be configured to measure or collect data of the vital sign of the user, for example, a mobile phone or a tablet computer.

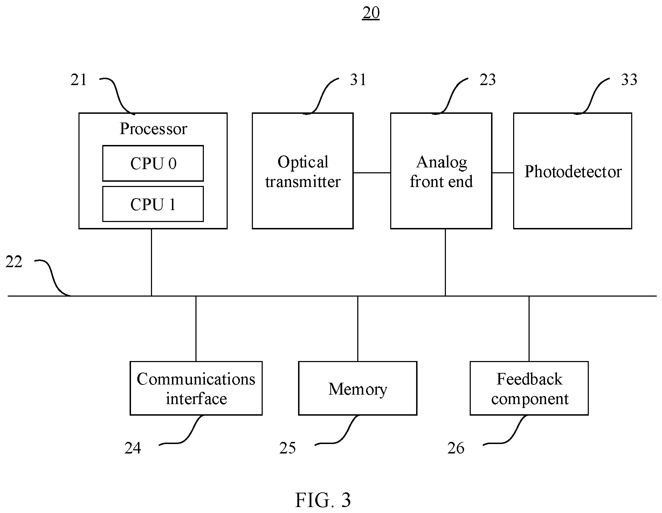

Refer to FIG. 3. A photosensitive apparatus 20 includes a processor 21, a communications bus 22, an analog front end (Analog Front End, AFE for short) 23, a communications interface 24, a memory 25, an optical transmitter 31, and a photodetector 33. The analog front end 23, the communications interface 24, and the memory 25 each establish a communication connection with the processor 21.

The analog front end 23 is connected to the optical transmitter 31 and the photodetector 33, and establishes a communication connection with the processor 21. The analog front end 23 is configured to: control generation of an optical signal of the optical transmitter 31, receive a signal output by the photodetector 33, convert the signal into a digital signal, and then transmit the digital signal to the processor 21 for processing, or convert the signal into a digital signal, perform corresponding processing (for example, digital filtering), and then transmit the digital signal to the processor 21 for processing.

Specifically, under control of the processor 21, the analog front end 23 drives the optical transmitter 31 to transmit a test optical signal to a skin of a user. After the test optical signal irradiates the skin of the user, some test optical signals enter the skin and is reflected and/or scattered, and some reflected and/or scattered light enters the photodetector 33 and is received by the photodetector 33. A reflected and/or scattered signal received by the photodetector 33 is referred to as a backward reflected and/or scattered signal, and is briefly referred to as a backward signal. The backward signal has varying intensity, and changes with a blood flow and a pulse inside a skin of a human body. The photodetector 33 converts the received backward signal into an electrical signal, and feeds the electrical signal back to the analog front end 23, the analog front end 23 performs digital conversion (the analog front end 23 may further perform corresponding digital processing, for example, digital filtering), and feeds back or transmits, to the processor 21, a digital signal obtained through conversion. The processor 21 is configured to perform processing and analysis on the digital signal fed back by the analog front end 23, to obtain a vital sign of the user.

The processor 21 may be a central processing unit (Central Processing Unit, CPU), or may be another general purpose processor, a digital signal processor (Digital Signal Processor, DSP), an application specific integrated circuit (Application Specific Integrated Circuit, ASIC), a field-programmable gate array (Field-Programmable Gate Array, FPGA), or another programmable logic device, discrete gate or transistor logic device, discrete hardware component, or the like. The general purpose processor may be a microprocessor, or the processor may be any conventional processor, or the like. The processor 21 is a control center of the photosensitive apparatus 20, and is connected to various parts of the entire photosensitive apparatus 20 through various interfaces and lines. The communications bus 22 may include a path, to transfer information between the foregoing components.

The communications interface 24, by using any apparatus of a transceiver type, is configured to communicate with another device or communications network such as Ethernet, a radio access network (radio access network, RAN for short), a wireless local area network (wireless local area networks, WLAN for short), a serial peripheral interface (Serial Peripheral Interface, SPI for short), or an internal integrated circuit bus (Inter-Integrated Circuit Bus, I2C for short). The processor 21 and each component may have a same communications interface 24 or may have different communications interfaces 24.

The memory 25 may be configured to store a computer program and/or a module. The processor 21 runs or executes the computer program and/or the module stored in the memory 25 and invokes data stored in the memory 25, to implement various functions of the photosensitive apparatus 20. The memory 25 may mainly include a program storage area and a data storage area. The program storage area may store an operating system, an application (for example, a sound playing function or an image playing function) that is required by a plurality of functions, and the like. The data storage area may store data (for example, audio data or a phone book) that is created based on use of the photosensitive apparatus 20, and the like. In addition, the memory 25 may include a high-speed random access memory, and may further include a nonvolatile memory, for example, a hard disk, a memory, an insertion-type hard disk, a smart media card (Smart Media Card, SMC), a secure digital (Secure Digital, SD) card, a flash card (Flash Card), a plurality of magnetic storage devices, a flash memory device, or another volatile solid-state storage device. The memory 25 may exist independently, and is connected to the processor 21 through a communication connection line. Alternatively, the memory 25 may be integrated with the processor 21. The term "and/or" describes only an association relationship for describing associated objects and represents that three relationships may exist. For example, A and/or B may represent the following three cases: Only A exists, both A and B exist, and only B exists. In addition, in the descriptions in embodiments of this application, "a plurality of" means two or more.

In a specific implementation, in an embodiment, the photosensitive apparatus 20 may include a plurality of processors 21 such as a CPU 0 and a CPU 1 in FIG. 3. Each of the processors 21 may be a single-core (single-CPU) processor or a multi-core (multi-CPU) processor. Herein, the processor may be one or more devices, circuits, and/or processing cores configured to process data (for example, computer program instructions).

In some implementations, the analog front end 23 may be omitted, and the processor 21 directly controls the optical transmitter 31, and processes and analyzes the electrical signal fed back by the photodetector 33, to further obtain the vital sign of the user. The optical transmitter 31 is configured to transmit a test optical signal to the skin of the user under control of the processor 21. After the test optical signal irradiates the skin of the user, some test optical signals enter the skin and is reflected and/or scattered, and some reflected and/or scattered light enters the photodetector 33 and is received by the photodetector 33. A reflected and/or scattered signal received by the photodetector 33 is referred to as a backward signal. The backward signal has varying intensity, and changes with a blood flow and a pulse inside the skin of the human body. The photodetector 33 converts the received backward signal into an electrical signal and feeds back the electrical signal to the processor 21. The processor 21 is configured to perform processing and analysis on the electrical signal fed back by the photodetector 33, to obtain the vital sign of the user.

The photosensitive apparatus 20 further includes a feedback component 26, configured to send an alarm to the user when the vital sign that is of the user and that is obtained by the processor 21 exceeds a preset threshold, for example, when a heart rate of the user exceeds a preset heart rate. In this implementation, the feedback component 26 is a speaker, and the processor 21 controls the feedback component 26 to send an audio alarm. It can be understood that the feedback component 26 may alternatively be a display, a vibrator, or the like. This is not limited herein.

### Implementation 1

In the conventional technology, a test light spot that corresponds to a test optical signal transmitted by a light source and that irradiates a skin of a user may be considered as an original light spot output by the light source. The original light spot includes an original light spot center and an edge region disposed around the original light spot center. Light intensity at the original light spot center is much greater than light intensity in the edge region. A test optical signal transmitted from the original light spot center is referred to as a central light spot optical signal. Most of test optical signals transmitted by the light source, especially the test optical signal from the original light spot center with high light intensity, are reflected by the skin back to the light source and cannot be used.

If utilization of the central light spot optical signal transmitted from the original light spot center is improved, utilization of the light source can be effectively improved, to reduce a loss of the light source. For this purpose, a photosensitive apparatus provided in Implementation 1 of this application adjusts a light intensity distribution of the original light spot, adjusts the central light spot optical signal to be scattered or shifted from the original light spot center to a direction away from the original light spot center, and leads more central light spot optical signals to the direction away from the original light spot center, so that the test light spot that is output by the light source and that irradiates the skin of the user is different from the original light spot, and a photodetector disposed around the light source can receive more effective backward signals.

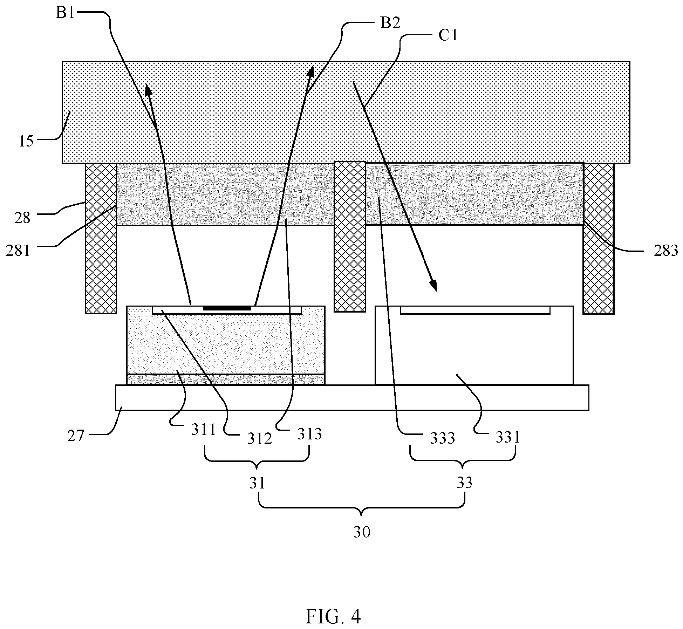

Refer to FIG. 4. A photosensitive apparatus 30 further includes a mount 27 and a housing 28, and the mount 27 is fixedly accommodated inside the housing 28. In this implementation, the mount 27 is a circuit board, and the mount 27 is a mainboard or a sub-board of the photosensitive apparatus 20. When the mount 27 is the mainboard of the photosensitive apparatus 20, a processor 21, a communications interface 24, and a memory 25 are fastened on the mount 27. Alternatively, when the mount 27 is the sub-board of the photosensitive apparatus 20, a processor 21, a communications interface 24, and a memory 25 are fastened on another mainboard. Alternatively, when the mount 27 is the sub-board of the photosensitive apparatus 20, one processor 21 is on the sub-board, and one or more processors 21, a communications interface 24, and a memory 25 are fastened on another mainboard. It can be understood that the mount 27 is not limited to the circuit board. The mount 27 may be another supporting structure. This is not limited herein. Alternatively, the mount 27 may be omitted. For example, the processor 21, the communications interface 24, the memory 25, an optical transmitter 31, and a photodetector 33 are directly fastened to the housing 28.

The housing 28 is provided with a first light-passing hole 281 and a second light-passing hole 283 that are disposed at an interval. The housing 28 is configured to protect a component such as the processor 21 accommodated inside the housing 28.

The optical transmitter 31 includes a light source 311 and a light exit window 313. The light source 311 is configured to output an original light spot, to transmit a test optical signal (B1 and B2 indicated in FIG. 4). The light source 311 is provided with a light exit structure 312, configured to transmit the test optical signal transmitted by the light source 311. The light exit window 313 is located on an optical path of the test optical signal. The light exit window 313 is fastened inside the first light-passing hole 281 of the housing 28. After the test optical signal sequentially passes through the light exit structure 312 and the light exit window 313, a test light spot is formed, and then irradiates the skin of the user. The photodetector 33 includes a detector body 331 and an optical receiving window 333. The detector body 331 is configured to receive a backward signal that is transmitted through the optical receiving window 333 (as shown by C1 in FIG. 4). The optical receiving window 313 is fastened inside the second light-passing hole 283.

In this implementation, the light source 311 is fastened on the mount 27, the light source 311 is a light source chip, and the light exit window 313 and the light exit structure 312 are disposed at an interval. It can be understood that the light exit window 313 and the light exit structure 312 may be fastened relative to each other, or the light source 311 and the light exit structure 312 may be separated and disposed separately. It can be understood that the light source 311 may be a separate photo-emitting layer. In other words, no cover is required, and no light exit structure 312 needs to be disposed. It can be understood that a structure of the light source 311 is not limited. It only needs to be ensured that the light source 311 can transmit a test optical signal. For example, the light source 311 includes a photo-emitting layer and a cover 3113, the photo-emitting layer is disposed at a bottom of the cover, and the light exit structure 312 is disposed on the cover, and is configured to transmit the test optical signal generated by the photo-emitting layer 3111.

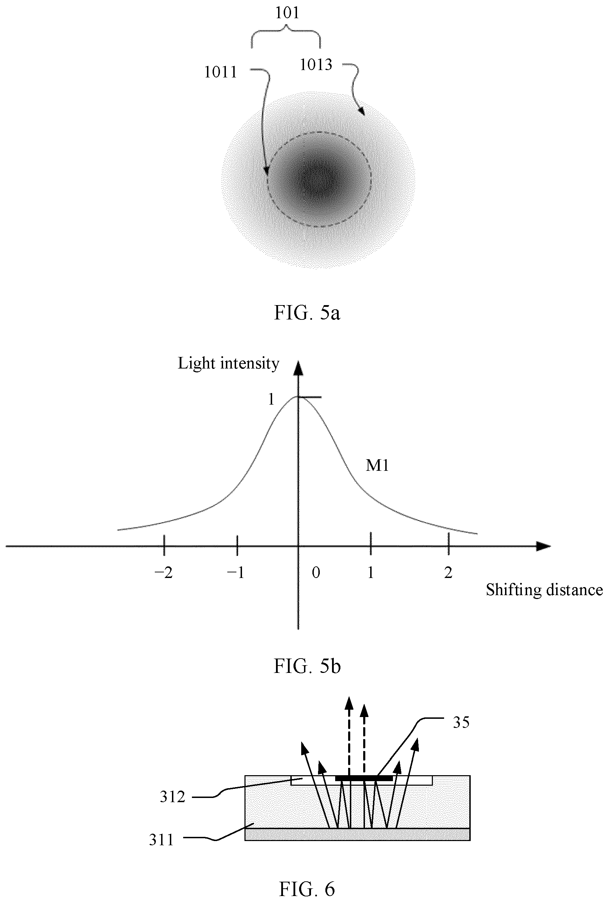

The original light spot output by the light source 311 may be considered as a centrosymmetric light spot. Refer to FIG. 5a. In this implementation, the original light spot is approximately in a circular shape. An original light spot 101 includes an original light spot center 1011 and an edge region 1013 disposed around the original light spot center 1011. Light intensity at the original light spot center 101 falls within a first light intensity range, light intensity in the edge region 1013 falls within a second light intensity range, and minimum light intensity in the first light intensity range is much greater than maximum light intensity in the second light intensity range. For example, the minimum light intensity in the first light intensity range and the maximum light intensity in the second light intensity range are not of a same order of magnitude, so that the original light spot center 1011 is obviously different from the edge region 1013. A test optical signal transmitted from the original light spot center 1011 is a central light spot optical signal.

There is maximum light intensity at the original light spot center, and a longer distance from the original light spot center leads to weaker light intensity. FIG. 5b is a schematic diagram of a light intensity distribution curve corresponding to FIG. 5a. An origin 0 represents the original light spot center, a longitudinal axis represents light intensity, and a lateral axis represents a distance from the original light spot center. A light intensity distribution curve M1 of the original light spot is a single-bell curve.

Refer to FIG. 6. The photosensitive apparatus 20 further includes a lens structure 35, configured to scatter or shift the central light spot optical signal in a direction away from the original light spot center, to convert the original light spot into a test light spot. The lens structure 35 is disposed on the light exit structure 312, and the central light spot optical signal is incident to the lens structure 35 by using the light exit structure 312. In this implementation, the lens structure 35 includes a grating structure, configured to diffract the incident central light spot optical signal, so that the central light spot optical signal is scattered in a direction away from the original light spot center, to convert the original light spot into a test light spot.

In this implementation, there is one light exit structure 312, and the light exit structure 312 is a transparent channel.

It can be understood that the lens structure 35 may alternatively be a reflective film, and the original light spot output by the light source 311 is adjusted by the lens structure 35 and is converted into a test light spot.

It can be understood that the light exit structure 312 may be a transparent hole, or the light exit structure 312 may be a transparent layer made of a transparent material.

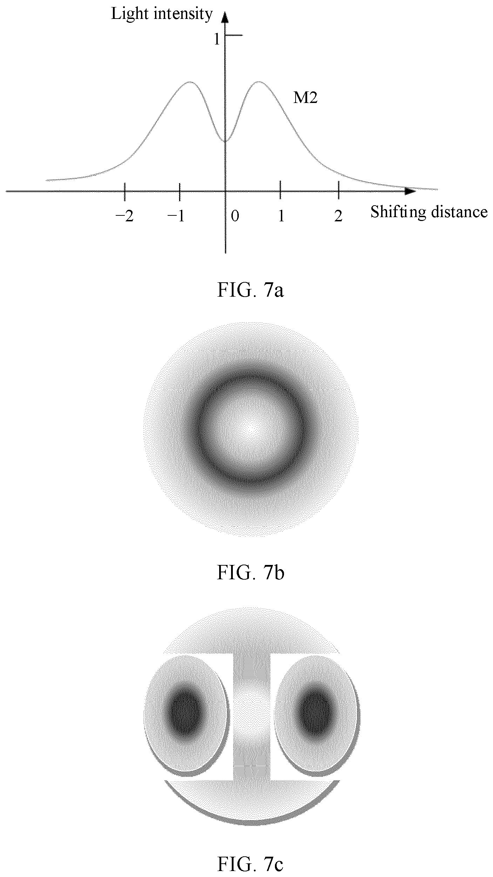

Due to an adjustment function of the lens structure 35, after the test optical signal transmitted by the light source 311 is transmitted through the light exit structure 312, the test optical signal is scattered in the direction away from the original light spot center. In other words, some test optical signals are scattered from a beam center to the direction away from the beam center, to re-adjust a light intensity distribution of the light spot. FIG. 7a is a schematic diagram of a light intensity distribution curve of a test light spot. A light intensity distribution curve M2 of the test light spot is a double-bell distribution curve. It can be learned that, in comparison with the original light spot, light intensity at a center of the test light spot is reduced, and light intensity of a part that is of the test light spot and that is away from the center of the test light spot is enhanced, so that the photodetector 33 can receive more effective backward signals, to reduce a light loss, and improve utilization of the light source 311.

The lens structure 35 is disposed, to obtain a required test light spot. For example, referring to FIG. 7b, the test light spot may be an annular light spot, the light intensity at the center of the test light spot is less than light intensity at a location around the center of the test light spot, and a darker color indicates higher light intensity in FIG. 7b.

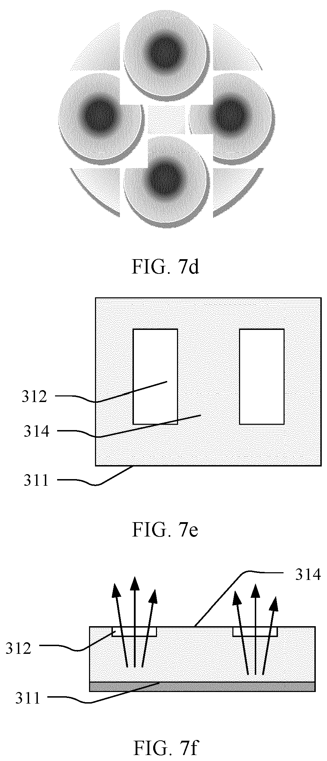

A shape of the test light spot may be different from that of the original light spot. For example, the original light spot is in a circular shape, and the test light spot is in an elliptic shape, or the like. The test light spot may be an N-point light spot (which may also be referred to as an N-center light spot), where N is greater than or equal to 2, for example, a double-point light spot (which may also be referred to as a double-center light spot as shown in FIG. 7c) or a four-point light spot (which may also be referred to as a four-center light spot as shown in FIG. 7d).

In another implementation, the shape of the test light spot may be adjusted by changing a quantity of light exit structures 312 of the optical transmitter 31. In other words, an adjustment structure includes a light exit structure 312. For example, referring to FIG. 7e and FIG. 7f, two light exit structures 312 are disposed at an interval in the light source 311. In other words, the two adjacent light exit structures 312 are connected by using a non-transparent light-shielding region 314, and the light-shielding regions 314 is located on an optical path of the central light spot optical signal, so that the central light spot optical signal is blocked and cannot be transmitted, and the test optical signal can only be transmitted from the two light exit structures 312, to adjust the original light spot to a double-point light spot (also referring to FIG. 7c).

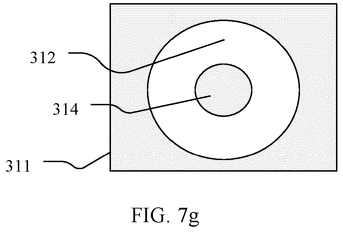

In another implementation, the shape of the test light spot may be adjusted by changing a shape of the light exit structure 312 of the optical transmitter 31. In other words, an adjustment structure includes the light exit structure 312. For example, referring to FIG. 7g, an annular light exit structure 312 is disposed around the light-shielding region 314, the light-shielding region 314 is located on an optical path of the central light spot optical signal, the central light spot optical signal is blocked and cannot be transmitted, and the test optical signal transmitted by the light source 311 can only be transmitted from the annular light exit structure 312, to form an annular test light spot (as shown in FIG. 7b).

It can be understood that the adjustment structure includes a light exit structure disposed on the light source, and the light exit structure is disposed by avoiding an original output optical path of the central light spot optical signal.

It can be understood that there are at least two light exit structures, and the two adjacent light exit structures are disposed at an interval, so that the test light spot is an N-point light spot, where N is greater than 1.

It can be understood that the light exit structure is an annular structure, and the test light spot is an annular light spot.

It can be understood that the original light spot output by the light source 311 is not limited to a centrosymmetric pattern, the original light spot may alternatively be an irregular pattern, and an optical signal transmitted from a region in which light intensity of the original light spot is high (including a region with maximum light intensity) is a central light spot optical signal.

### Implementation 2

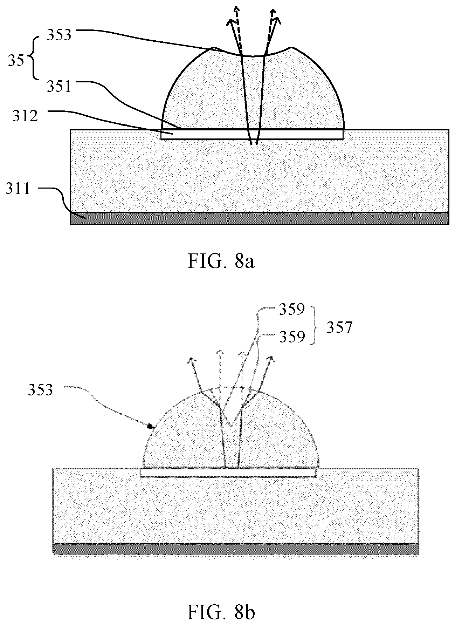

An optical transmitter provided in Implementation 2 of this application is approximately the same as the optical transmitter provided in Implementation 1. A difference lies in that an adjustment structure includes a lens structure. FIG. 8a to FIG. 8h are schematic diagrams of possible structures of an optical transmitter according to Implementation 2. A dashed line arrow indicates a transmission direction of a central light spot optical signal after the central light spot optical signal is transmitted from a light exit structure in a conventional photosensitive apparatus, and a solid line arrow indicates a transmission direction of a central light spot optical signal after the central light spot optical signal passes through the adjustment structure in the conventional photosensitive apparatus provided in Implementation 2.

A structure shown in FIG. 8a is briefly described below. A lens structure 35 includes an incident side 351 and an emergent side 353. The incident side 351 is disposed toward a light source 311 and is fastened on a light exit structure 312. It can be understood that the incident side 351 may not be fastened on the light exit structure 312, and it only needs to be ensured that there is no gap between the incident side 351 and the light exit structure 312. A recess 357 that is concavely disposed toward a side on which the light source 311 is located is formed on the emergent side 353 of the lens structure 35. The central light spot optical signal is incident to the lens structure 35 from an orthogonal projection coverage of the recess 358 on the incident side 351, and is transmitted from the recess 357. In this implementation, the emergent side 353 further includes a spherical surface protruding in a direction away from the light source 311, to converge edge light transmitted from an edge region of an original light spot.

In this implementation, it is assumed that an output optical path on which a test optical signal is transmitted from the original light spot and does not pass through another element or component is an original output optical path. The recess 357 is a spherical surface that sinks toward the light source 311, and a lowest point of the recess 357 is located on an original output optical path of a central light spot optical signal transmitted from an original light spot center.

The central light spot optical signal is incident to the lens structure 35 from the orthogonal projection coverage of the recess 358 on the incident side 351, is refracted by the lens structure 35, and then is transmitted from the emergent side 353. Under an adjustment action of the recess 357, the central light spot optical signal is scattered in a direction away from the original light spot center. The emergent side 353 can converge the edge light transmitted from the original light spot, to reduce a scattering angle of the entire test optical signal, so as to further help improve energy utilization of the light source of the optical transmitter.

Refer to FIG. 8b. The recess 357 on the emergent side 353 may include a slope 359, or the recess 357 may be a three-dimensional conical surface. The slope 359 is a projection of a three-dimensional conical surface. In other words, a longitudinal section of the recess 357 is in a cone shape. Based on a total internal reflection principle, a transmission direction of a test optical signal reaching the slope 359 is changed, to convert the original light spot into a test light spot. For example, the central light spot optical signal is incident from the incident side 351 to the slope 359, and is transmitted, through total internal reflection of the slope 359, from a remaining area on the emergent side 353 other than the recess 357.

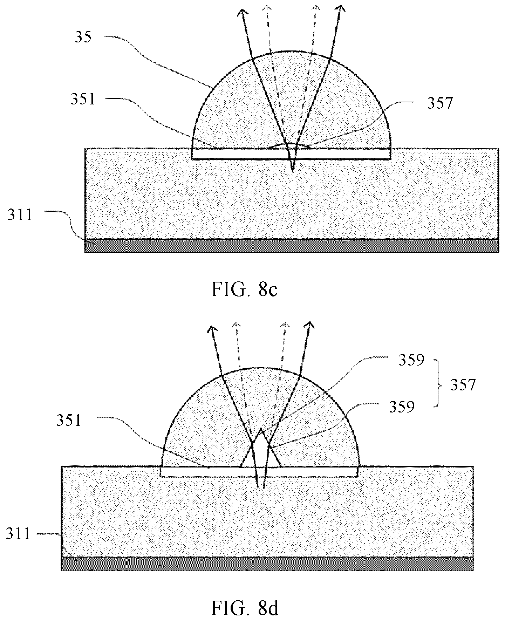

Refer to FIG. 8c. The recess 357 may be disposed on the incident side 351. The recess 357 is a spherical surface that is concavely disposed in a direction away from the light source 311. An incident angle at which the central light spot optical signal is incident to the lens structure 35 is changed, to change a refraction direction of the central light spot optical signal in the lens structure 35, so as to convert the original light spot into a test light spot.

Refer to FIG. 8d. The recess 357 may be disposed on the incident side 351, and the recess 357 is concavely disposed in a direction away from the optical transmitter 31. The recess 357 includes a slope 359 that is disposed through connection, or the recess 357 may be a three-dimensional conical surface, and the slope 359 is a projection of the three-dimensional conical surface. In other words, the longitudinal section of the recess 357 is in a cone shape. The incident angle at which the test optical signal of the light source 311 is incident to the lens structure 35 is changed by using the slope 359, to change the refraction direction, so as to convert the original light spot into a test light spot. For example, the central light spot optical signal is scattered in a direction away from the original light spot center after passing through the lens structure 35.

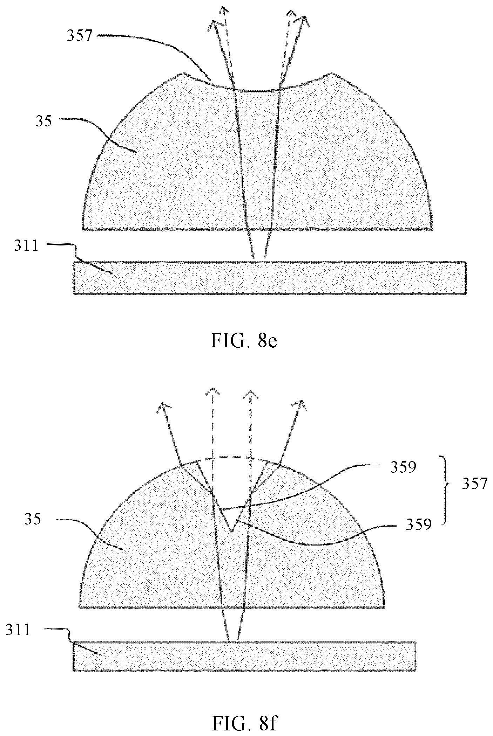

A structure shown in FIG. 8e is approximately the same as that shown in FIG. 8a. The recess 357 is a spherical surface that is concavely disposed toward the light source 311 of the optical transmitter 31. A difference lies in that the lens structure 35 and the light source 311 are disposed at an interval.

A structure shown in FIG. 8f is approximately the same as that shown in FIG. 8b. The recess 357 includes two slopes 359 disposed through connection, and the recess 357 is concavely disposed in a direction of the light source 311. A difference lies in that the lens structure 35 and the light source 311 are disposed at an interval. Based on a total internal reflection principle, a transmission direction of the central light spot optical signal of the original light spot is changed, to convert the original light spot into a test light spot.

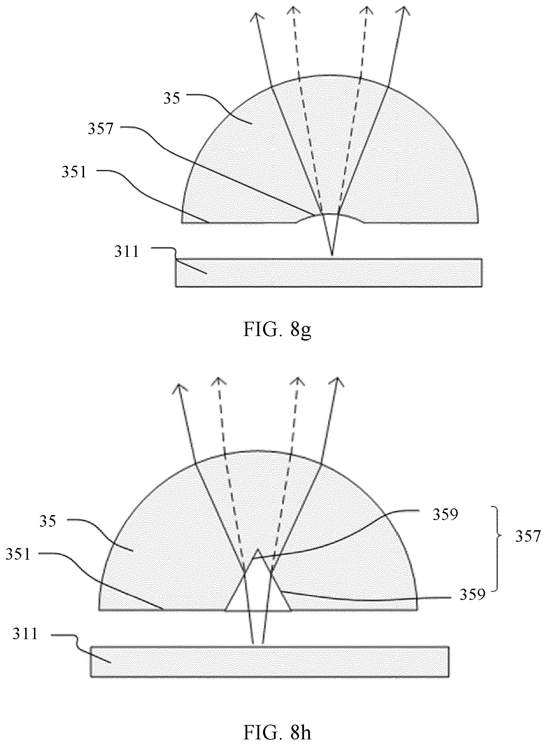

A structure shown in FIG. 8g is approximately the same as that shown in FIG. 8c. The recess 357 is disposed on the incident side 351, and the recess 357 is a spherical surface concavely disposed in the direction away from the light source 311. A difference lies in that the lens structure 35 and the light source 311 are disposed at an interval.

A structure shown in FIG. 8h is approximately the same as that shown in FIG. 8d. The recess 357 is disposed on the incident side 351, the recess 357 is a recess concavely disposed in a direction away from the light exit structure 312 and the light source 311, and the recess 357 includes a slope 359. A difference lies in that the lens structure 35 and the light source 311 are disposed at an interval.

It can be understood that the lens structure 35 of a lens type is not limited to the structures limited in FIG. 8a to FIG. 8h. For example, the lens structure 35 may be a combination of a plurality of lenses.

### Implementation 3

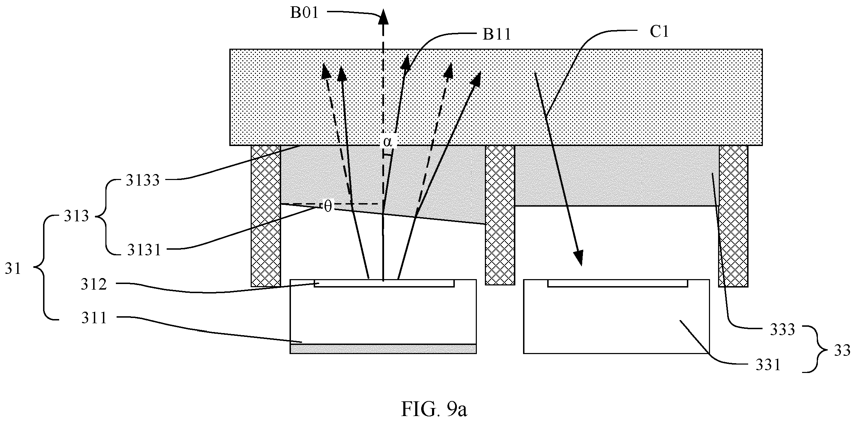

A photosensitive apparatus provided in Implementation 3 of this application is approximately the same as the photosensitive apparatus provided in Implementation 1. A difference lies in that an adjustment structure includes a light exit window. FIG. 9a to FIG. 9d are schematic diagrams of possible structures of a photosensitive apparatus according to Implementation 3. A dashed line arrow indicates a transmission direction of a central light spot optical signal after the central light spot optical signal passes through a light exit window in a conventional photosensitive apparatus, and a solid line arrow indicates a transmission direction of a central light spot optical signal after the central light spot optical signal passes through the light exit window in the photosensitive apparatus provided in Implementation 3.

Refer to FIG. 9a. An optical transmitter 31 and a photodetector 33 are disposed at an interval, the light exit window 313 includes a first interface 3131 and a second interface 3133 that are disposed opposite to each other, and the first interface 3131 is disposed toward a side of a light source 311. The second interface 3133 is a plane (a horizontal plane shown in FIG. 9a), so that the second interface 3133 is in close contact with a skin of a user, to prevent ambient light from entering the second interface 3133 to cause interference.

The first interface 3131 is a slope. In this implementation, the first interface 3131 is a plane. An included angle between a normal line of the first interface 3131 and a first direction is a first included angle, the first included angle is an acute angle, and the first direction is perpendicular to an original direction in which the central light spot optical signal is transmitted from an original light spot. The first interface 3131 inclines toward a direction in which a detector body 331 is located. In other words, a thickness of an end that is of the light exit window 313 and that is close to the photodetector 33 is greater than a thickness of an end away from the photodetector 33, to adjust an incident angle at which a test optical signal transmitted from the light source 311 is incident to the light exit window 313, and convert the original light spot into a test light spot, so that a transmission direction of a test optical signal transmitted from the second interface 3133 inclines toward the direction in which the detector body 331 is located, and more test optical signals are led to a side on which the photodetector 33 is located. The first interface 3131 is in contact with air.

It can be understood that the first interface 3131 is not limited to an interface in contact with air, and the first interface 3131 may be in contact with the light exit structure 312 or the light source 311 without a gap, or another medium exists between the first interface 3131 and the light exit structure 312. In only needs to be ensured that an incident angle of the central light spot optical signal can be reduced. The second interface 3133 is not limited to a plane. For example, the second interface 3133 may be a concave surface that is concavely disposed on a side that is of the light exit window 313 and that faces the optical transmitter 31, to achieve optimal coupling with the skin of the user. Certainly, the second interface 3133 may alternatively be set to a convex surface that is concavely disposed toward a direction away from a side on which the optical transmitter 31 is located.

A test optical signal transmitted from the light exit structure 312 is incident to the light exit window 313 through the first interface 3131, and then is transmitted from the second interface 3133. In this implementation, a refractive index of the light exit window 313 is 1.4, and a refractive index of air is 1. Because the first interface 3131 is a slope, the test optical signal transmitted from the second interface 3133 is deflected and inclines from an original transmission direction to the side on which the photodetector 33 is located. It is equivalent to that the existing first interface that is a plane rotates clockwise by an angle θ (in other words, the normal line of the first interface that is a plane rotates clockwise by the angle 0).

The central light spot optical signal is used as an example. If the first interface remains as a conventional plane disposed extending in the first direction, the central light spot optical signal is transmitted along a normal line direction of the second interface 3133 (B01 shown in FIG. 9a). In other words, the original light spot is approximately the same as the test light spot.

In this implementation, because the first interface 3131 is a slope, a central light spot optical signal of the original light spot is incident to the light exit window 313 after an original incident angle of 0 degrees (an angle between the central light spot optical signal and a normal line perpendicular to an incident surface) changes to an incident angle of θ, is refracted, is deflected by a deflection angle α in a rotation direction of the normal line, and then is transmitted from the second interface 3133, to adjust the original light spot by using the light exit window 313, to form a test light spot. A deflection angle at which the central light spot optical signal (B11 shown in FIG. 9a) is deflected toward a side of the detector body 331 is α, so that more effective backward signals C1 can be received by the photodetector 33.

Incident light at another angle rotates by an angle α in the rotation direction of the normal line (namely, in a direction of the photodetector 33), and a rotation angle is related to an incident angle of a ray of light. In this manner, more test optical signals of a central light spot part and an entire light source can be reflected and/or scattered by a skin, to enter the photodetector 33, so that the test optical signals are received by the detector body 331.

The original light spot may be considered as a centrosymmetric light spot. The original light spot center is used as a reference. A transmission direction of a test optical signal on a side that is of the original light spot and that is close to the photodetector deviates toward the photodetector 33, and a transmission direction of a test optical signal on a side that is of the original light spot and that is away from the photodetector 33 deviates away from the photodetector 33. To lead more test optical signals to the photodetector 33, the test optical signal on the side that is of the original light spot and that is away from the photodetector 33 may be adjusted by a greater angle.

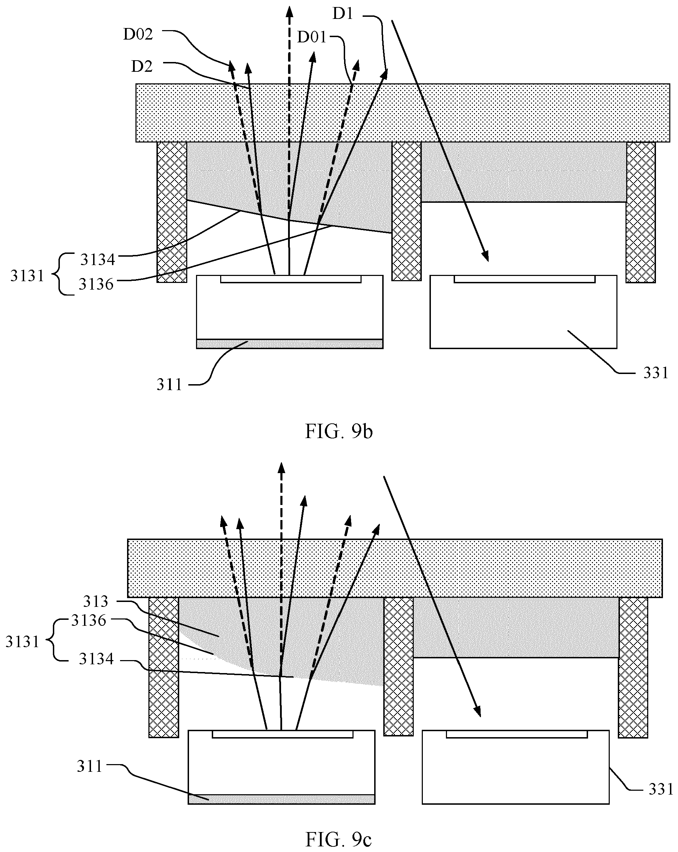

In an implementation, referring to FIG. 9b, the first interface 3131 includes a first connection surface 3134 and a second connection surface 3136 that are disposed through connection, and the first connection surface 3134 is disposed close to the detector body 331. Both the first connection surface 3134 and the second connection surface 3136 are inclined surfaces inclining toward the direction in which the detector body 331 is located, an included angle between a normal line of the second connection surface and the first direction is a second included angle, and the second included angle is greater than the first included angle. In this implementation, the first connection surface 3134 and the second connection surface 3136 are planes, and a slope of the first connection surface 3134 is less than a slope of the second connection surface 3136. A connection point between the first connection surface 3134 and the second connection surface 3136 is located on an original output optical path of the central light spot optical signal transmitted from the original light spot center, so that more test optical signals on the side that is of the original light spot and that is away from the photodetector 33 are led to the side on which the detector body 331 is located. The central light spot optical signal is incident from the connection point between the first connection surface 3134 and the second connection surface 3136. However, an entire region of the first interface shown in FIG. 9a is the first connection surface.

It is assumed that a test optical signal that is incident from the first connection surface 3134 and then is transmitted from the second interface 3133 is a first test optical signal D1, D01 is an original transmission direction of the first test optical signal in a conventional light exit window, in other words, a transmission direction existing when a conventional first interface corresponding to the first test optical signal is a plane disposed extending along the first direction, and a deflection angle of the first test optical signal is a first deflection angle. It is assumed that light that is incident from the second connection surface 3136 and then is transmitted from the second interface 3133 is a second test optical signal D2, D02 is an original transmission direction of the second test optical signal in the conventional light exit window, in other words, a transmission direction existing when a conventional first interface corresponding to the second test optical signal is disposed extending along the first direction, and a deflection angle of the second test optical signal is a second deflection angle. The first deflection angle is greater than the second deflection angle, so that more test optical signals on the side that is of the original light spot and that is away from the photodetector 33 are led to the side on which the photodetector 33 is located.

It can be understood that the second connection surface 3136 is not limited to a plane. Refer to FIG. 9c. The first connection surface 3134 remains as an inclined plane, the second connection surface 3136 may be a curved surface that is concavely disposed from a side that is of the light exit window 313 and that faces the light source 311, and the second connection surface 3136 is disposed approximately inclining toward the direction in which the detector body 331 is located, to obtain a larger deflection angle, and further help adjust, to the direction of the photodetector 33, the test optical signal transmitted by the light source 311.

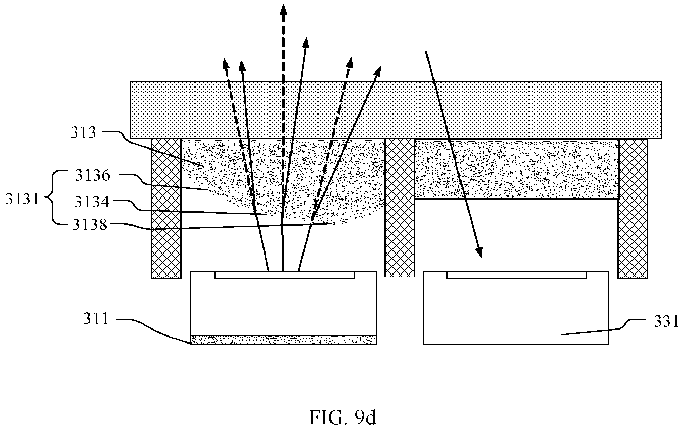

It can be understood that, referring to FIG. 9d, the first interface 3131 further includes a third connection surface 3138, the first connection surface 3134 is located between the second connection surface 3136 and the third connection surface 3138, the third connection surface 3138 is located on a side that is of the first connection surface 3134 and that is close to the photodetector 33, the first connection surface 3134 is a slope, the second connection surface 3136 and the third connection surface 3138 each are an arc surface that is concavely disposed from a side that is of the light exit window 313 and that faces the light source 311, and the third connection surface 3138 can be used to reduce a quantity of test optical signals that are transmitted from the optical transmitter, that are close to the detector body 331, whose angles exceed a specific angle, and that are incident to a housing.

It can be understood that a structure of the first interface 3131 is not limited, and the first interface 3131 may be a combination of a plane, a slope, a curved surface, or another type of curved surface, to adjust an optical path, adjust more test optical signals sent by the optical transmitter 31 toward a side of the photodetector 33, and enhance intensity of a backward signal that may be received by the photodetector 33, thereby improving utilization of the light source, and improving performance of a photosensitive apparatus 20.

The foregoing describes an optical path structure (which is assumed to be an X direction) along a cross section of a center of the optical transmitter and a center of the photodetector, and an optical path may be disposed in a similar manner on a cross section (a Y direction) perpendicular to a current cross section. If there is no photodetector on the cross section perpendicular to the current cross section, light transmitted by the light source may be adjusted (converged) toward a center of the light source by using two curved surfaces on the cross section perpendicular to the current cross section. This is not limited herein.

### Implementation 4

In a conventional technology, not all light reaching an optical receiving window can be received by a photodetector. When an incident angle at which a backward signal is incident to the optical receiving window is greater than a specific value, for example, when the incident backward signal is parallel to an incident surface of the optical receiving window, the backward signal cannot be received by the photodetector. In addition, a significant feature of photodetection is that a larger incident angle leads to lower photoelectric conversion efficiency. In other words, when the backward signal is perpendicularly incident to the photodetector, there is highest photoelectric conversion efficiency. Therefore, a smaller incident angle at which the backward signal is incident to the photodetector leads to more convenient receiving of the photodetector.

A photosensitive apparatus provided in Implementation 4 of this application aims to minimize, as much as possible, an incident angle at which a backward signal is incident from the optical receiving window to a detector body.

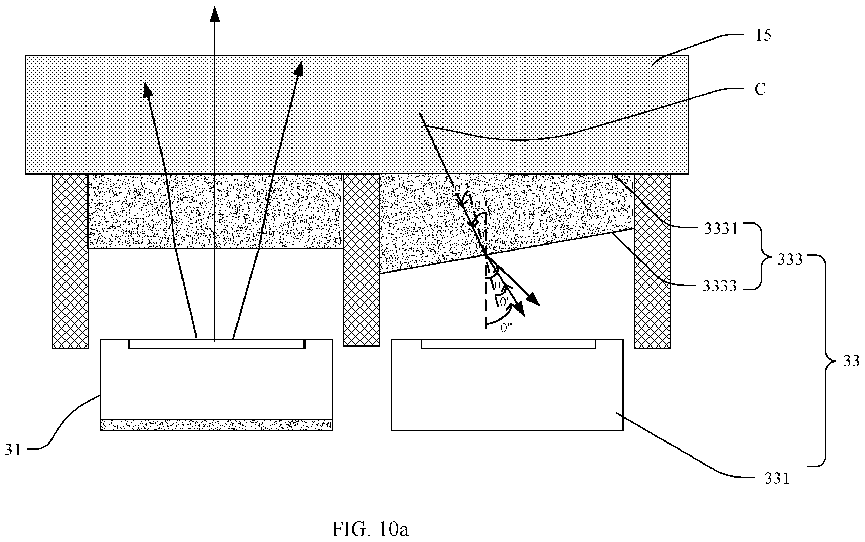

Specifically, referring to FIG. 10a, a photodetector 33 includes a detector body 331 and an optical receiving window 333. The optical receiving window 333 includes a first surface 3331 and a second surface 3333 that are disposed opposite to each other, and the second surface 3333 is disposed on a side that is of the optical receiving window 333 and that faces the detector body 331.

In this implementation, the first surface 3331 is a plane, so that the first surface 3331 is in close contact with a skin of a user, to prevent ambient light from entering the first surface 3331 to cause interference. It can be understood that the first surface 3331 is not limited to a plane. For example, the first surface 3331 may be a concave surface that is concavely disposed in a direction of a side that is of the optical receiving window 333 and that faces the detector body 331, to achieve optimal coupling with the skin of the user. Certainly, the first surface 3331 may alternatively be set as a convex surface that is concavely disposed toward a direction away from a side on which the detector body 331 is located.

The second surface 3333 is a slope, the second surface 3333 inclines toward the first surface 3331, and a thickness of the optical receiving window 333 decreases from an end that is of the optical receiving window 333 and that is close to the optical transmitter 31 to an end away from the optical transmitter 31. The second surface 3333 is configured to reduce an incident angle at which the backward signal is incident to the detector body 331.

In this implementation, a refractive index of the optical receiving window 333 is 1.4, and a refractive index of air is 1. Because the second surface 3333 is a slope, a backward signal transmitted from the second surface 3333 approaches a normal line of the first surface 3331 from an original transmission direction, to further reduce the incident angle at which the backward signal is incident to the detector body 331.

It is equivalent to rotate, counterclockwise by a specific angle, a first interface in which a side that is of the conventional optical receiving window and that faces the optical transmitter intersects with air. The second surface 3333 is set to be a slope. A backward signal C entering the optical receiving window 333 from the skin 15 of the user through the first surface 3331 is used as an example. An incident angle at which the backward signal C reaches the second surface 3333 decreases from α to α', and then, an incident angle at which the backward signal C is incident to the detector body 331 decreases from θ to θ". In this manner, the detector body 331 can receive more backward signals, and conversion efficiency of the photodetector 33 can be improved.

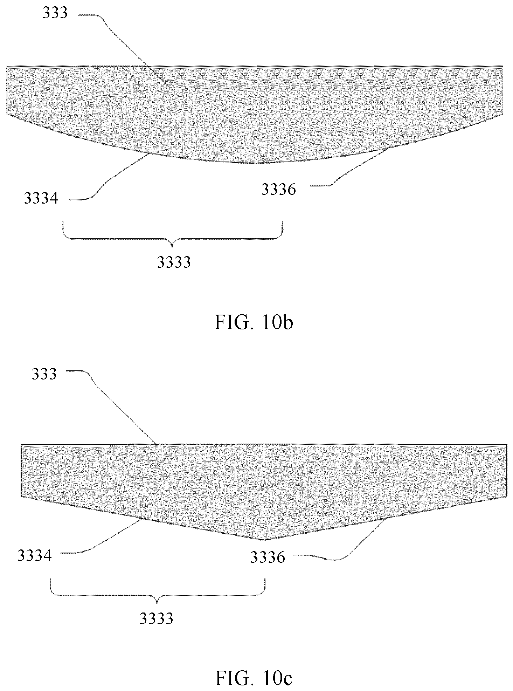

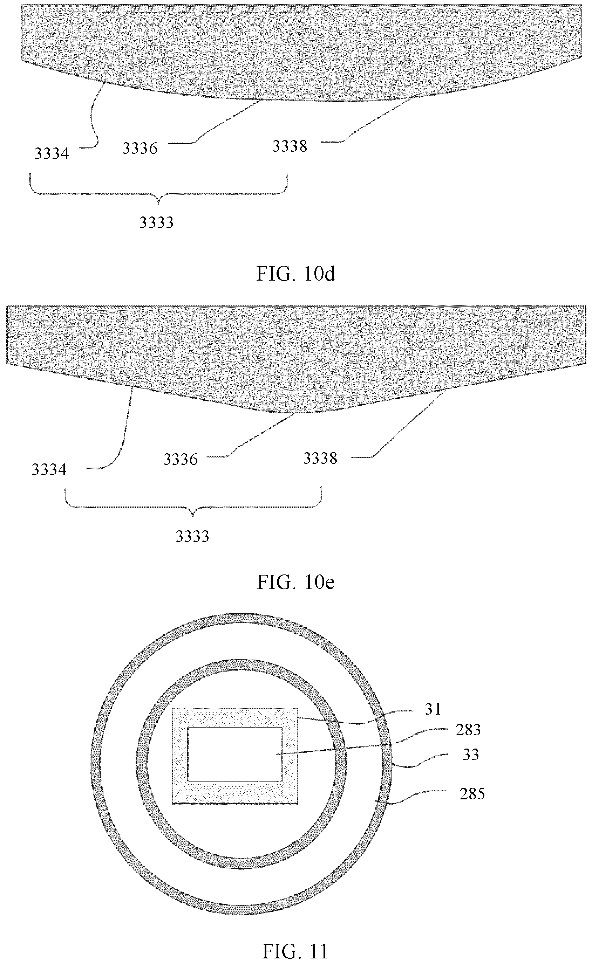

It can be understood that the second surface 3333 is not limited to be a slope, and the second surface 3333 may be in another shape to change an incident direction in which the backward signal is incident to the detector body 331. For example, the second surface 3333 is a curved surface that is concavely disposed from a side that is of the optical receiving window 333 and that faces the photodetector 33. As shown in FIG. 10b, the second surface 3333 includes a first sub-surface 3334 and a second sub-surface 3336 that are disposed through connection, the first sub-surface 3334 is disposed close to the optical transmitter 31, and the first sub-surface 3334 and the second sub-surface 3336 each are a curved surface. As shown in FIG. 10c, the second surface 3333 includes a first sub-surface 3334 and a second sub-surface 3336 that are disposed through connection, and the first sub-surface 3334 and the second sub-surface 3336 each are a slope. As shown in FIG. 10d, the second surface 3333 includes a first sub-surface 3334, a second sub-surface 3336, and a third sub-surface 3338 that are sequentially disposed through connection, the first sub-surface 3334 and the third sub-surface 3338 each are a curved surface, and the second sub-surface 3336 is a slope. As shown in FIG. 10e, the second surface 3333 includes a first sub-surface 3334, a second sub-surface 3336, and a third sub-surface 3338 that are sequentially disposed through connection, the first sub-surface 3334 and the third sub-surface 3338 each are a slope, and the second sub-surface 3336 is a curved surface. FIG. 10a to FIG. 10e merely show an example shape structure of the second surface 3333. The second surface 3333 may alternatively be any combination of a curved surface, a slope, and a curved surface.

### Implementation 5

In a conventional photosensitive setting for which a PPG technology is used, a photodetector is usually disposed on one side of a light source or a plurality of photodetectors are disposed around an optical transmitter, and processing operation space needs to be reserved between photodetectors, so that when a backward signal returns to a gap between adjacent photodetectors, the backward signal cannot be received by a photodetector.

In order that more effective backward signals can be detected, in Implementation 5 of this application, as shown in FIG. 11, a photodetector 33 has an annular structure, an optical receiving window 333 has an annular structure, an optical transmitter 31 is located at a center of the photodetector 33, and the center of the photodetector 33 overlaps a center of the optical receiving window 333. All backward signals at a specific distance from the center within an annular range can be received by the photodetector 33, to improve utilization of a light source, improve performance of a photosensitive apparatus, or reduce power consumption of a photosensitive apparatus.

It can be understood that it is not specified that the optical transmitter 31 is located on the center of the photodetector 33, the center of the photodetector 33 overlaps the center of the optical receiving window 333, and it only needs to be ensured that the photodetector 33 is disposed around the optical transmitter.

It can be understood that, in an implementation, when there is no conflict, the photosensitive apparatus may include at least one of the adjustment structures in Implementation 1 to Implementation 5. For example, the photosensitive apparatus includes an optical transmitter and a photodetector, the optical transmitter includes a light source and a light exit window, a grating and/or a reflective film are/is disposed on a light exit structure of the light source, a first connection surface is disposed on a side that is of the light exit window and that faces the light source, the photodetector includes an optical receiving window and a photodetector body, an inclined surface is disposed inclining toward the light source is disposed on a second surface that is of the optical receiving window and that faces the detector body, both the detector body and the optical receiving window are of an annular structure, and both the detector body and the optical receiving window are disposed around the optical transmitter.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. An optical transmitter (31) used for photoplethysmography, comprising a light source (311) and an adjustment structure, wherein the light source (311) is configured to output an original light spot, to transmit a test optical signal to a skin of a user, the adjustment structure is located on an output optical path of the test optical signal, a test optical signal transmitted from an original light spot center of the original light spot is a central light spot optical signal, and the adjustment structure is configured to scatter the central light spot optical signal in a direction away from the original light spot center, to convert the original light spot into a test light spot, wherein the optical transmitter (31) further comprising a light exit structure (312) disposed on the light source (311), wherein the central light spot optical signal is incident to the adjustment structure by using the light exit structure (312), and wherein the adjustment structure comprises a lens structure (35), the lens structure (35) comprises an incident side (351) and an emergent side (353), the incident side is disposed toward the light source (311), a recess that is concavely disposed toward a side on which the light source (311) is located is disposed on the emergent side (353) of the lens structure (35), and the central light spot optical signal is incident to the lens structure (35) from an orthogonal projection coverage of the recess on the incident side, and wherein the emergent side (353) further includes a spherical surface protruding in a direction away from the light source (311), to converge edge light transmitted from an edge region of an original light spot.

2. The optical transmitter (31) according to claim **1,** wherein the adjustment structure comprises a grating structure, configured to diffract the incident central light spot optical signal; and/or the adjustment structure comprises a reflective film, configured to reflect the incident central light spot optical signal.

3. The optical transmitter (31) according to claim **1,** wherein the adjustment structure comprises a lens structure (35), the lens structure (35) comprises an incident side and an emergent side, the incident side is disposed toward the light source (311), a recess that is concavely disposed toward a side away from the light source (311) is disposed on the incident side of the lens structure, and the central light spot optical signal can be incident to the lens structure (35) from the recess and be transmitted from the emergent side.

4. The optical transmitter (31) according to claim 1 or 3, wherein the incident side is fastened to the light exit structure (312).

5. The optical transmitter according to claim 1 or 3, wherein the emergent side comprises a spherical surface protruding in a direction away from the optical transmitter.

6. The optical transmitter according to claim 1, wherein the adjustment structure comprises a light exit structure (312) disposed on the light source (311), and the light exit structure (312) is disposed by avoiding an original output optical path of the central light spot optical signal.

7. The optical transmitter (31) according to claim 6, wherein there are at least two light exit structures (312), and two adjacent light exit structures are disposed at an interval, so that the test light spot is an N-point light spot, wherein N is greater than 1.

8. The optical transmitter (31) according to claim 6, wherein the light exit structure (312) is an annular structure, and the test light spot is an annular light spot.

9. The optical transmitter (31) according to claim 1, wherein the adjustment structure comprises a light exit window (313), the light exit window (313) comprises a first connection surface (3134), an included angle between a normal line of the first connection surface (3134) and a first direction is a first included angle, the first included angle is an acute angle, the central light spot optical signal is incident from the first connection surface (3134) to the light exit window (313), and the first direction is perpendicular to an original direction in which the central light spot optical signal is transmitted from the original light spot.

10. The optical transmitter (31) according to claim 9, wherein the light exit window (313) further comprises a second connection surface (3136) disposed by being connected to the first connection surface (3134), the first connection surface (3134) is disposed close to the photodetector (33) , an included angle between a normal line of the second connection surface (3136) and the first direction is a second included angle, and the second included angle is greater than the first included angle.

11. The optical transmitter (31) according to claim 10, wherein the light exit window (313) further comprises a third connection surface (3138), the third connection surface (3138) is connected to one end that is of the first connection surface (3134) and that is away from the second connection surface (3136), an included angle between a normal line of the third connection surface (3138) and the first direction is a third included angle, the second included angle is an acute angle, and the third included angle is an obtuse angle.

12. A photosensitive apparatus (20), comprising the optical transmitter (31) and the photodetector (33) according to any one of claims 1 to 11, wherein the photodetector (33) is configured to receive a backward signal obtained after a test optical signal is reflected and/or scattered by a skin of a user, to collect a vital sign of the user.

13. The photosensitive apparatus (20) according to claim 12, wherein the photodetector (33) comprises a detector body (331) and an optical receiving window (333), the backward signal enters the detector body (331) through the optical receiving window, the optical receiving window (333) comprises a first surface (3331) and a second surface (3333), the second surface (3333) is disposed toward the detector body (331), and the second surface (3333) comprises an inclined surface inclined towards a direction in which a light source (311) is located, to reduce an incident angle at which a backward signal transmitted from the second surface is incident to the detector body (331).

## Patentansprüche

1. Optischer Sender (31), der für Photoplethysmographie verwendet wird, umfassend eine Lichtquelle (311) und eine Justierstruktur, wobei die Lichtquelle (311) dazu konfiguriert ist, einen Originallichtfleck auszugeben, um ein optisches Testsignal auf eine Haut eines Benutzers auszusenden, wobei sich die Justierstruktur in einem optischen Ausgabeweg des optischen Testsignals befindet, ein optisches Testsignal, das von einem Originallichtfleckzentrum des Originallichtflecks ausgesendet wird, ein optisches Testsignal des zentralen Lichtflecks ist und die Justierstruktur dazu konfiguriert ist, das optische Testsignal des zentralen Lichtflecks in eine Richtung weg von dem Originallichtfleckzentrum zu streuen, um den Originallichtfleck in einen Testlichtfleck umzuwandeln, wobei der optische Sender (31) ferner eine Lichtaustrittsstruktur (312) umfasst, die an der Lichtquelle (311) angeordnet ist, wobei das optische Signal des zentralen Lichtflecks unter der Verwendung der Lichtaustrittsstruktur (312) auf die Justierstruktur einfällt und wobei die Justierstruktur eine Linsenstruktur (35) umfasst, die Linsenstruktur (35) eine Einfallsseite (351) und eine Ausfallsseite (353) aufweist, die Einfallsseite zu der Lichtquelle hin angeordnet ist (311), eine Vertiefung, die konkav zu einer Seite hin angeordnet ist, an der sich die Lichtquelle (311) befindet, auf der Ausfallsseite (353) der Linsenstruktur (35) angeordnet ist und das optische Signal des zentralen Lichtflecks von einer orthogonalen Projektionsabdeckung der Vertiefung auf der Einfallsseite auf die Linsenstruktur (35) einfällt und wobei die Ausfallsseite (353) ferner eine sphärische Oberfläche beinhaltet, die in eine Richtung weg von der Lichtquelle (311) vorsteht, um Randlicht, das von einem Randbereich eines Originallichtflecks ausgesendet wird, zu bündeln.

2. Optischer Sender (31) nach Anspruch 1, wobei die Justierstruktur eine Gitterstruktur umfasst, die dazu konfiguriert ist, das einfallende optische Signal des zentralen Lichtflecks zu beugen; und/oder wobei die Justierstruktur einen reflektierenden Film umfasst, der dazu konfiguriert ist, das einfallende optische Signal des zentralen Lichtflecks zu reflektieren.

3. Optischer Sender (31) nach Anspruch 1, wobei die Justierstruktur eine Linsenstruktur (35) umfasst, die Linsenstruktur (35) eine Einfallsseite und eine Ausfallsseite umfasst, die Einfallsseite zu der Lichtquelle (311) hin angeordnet ist, eine Vertiefung, die konkav zu einer Seite hin angeordnet ist, die von der Lichtquelle (311) abgewandt ist, auf der Einfallsseite der Linsenstruktur angeordnet ist und das optische Signal des zentralen Lichtflecks von der Vertiefung auf die Linsenstruktur (35) einfallen kann und von der Ausfallsseite ausgesendet werden kann.

4. Optischer Sender (31) nach Anspruch 1 oder 3, wobei die Einfallsseite an der Lichtaustrittsstruktur (312) befestigt ist.

5. Optischer Sender nach Anspruch 1 oder 3, wobei die Ausfallsseite eine sphärische Oberfläche umfasst, die in eine Richtung weg von dem optischen Sender vorsteht.

6. Optischer Sender nach Anspruch 1, wobei die Justierstruktur eine Lichtaustrittsstruktur (312) umfasst, die auf der Lichtquelle (311) angeordnet ist, und die Lichtaustrittsstruktur (312) durch Vermeiden eines originalen optischen Ausgabewegs des optischen Signals des zentralen Lichtflecks angeordnet ist.

7. Optischer Sender (31) nach Anspruch 6, wobei mindestens zwei Lichtaustrittsstrukturen (312) vorhanden sind und zwei benachbarte Lichtaustrittsstrukturen mit einem Intervall angeordnet sind, so dass der Testlichtfleck ein N-Punkt-Lichtfleck ist, wobei N größer als 1 ist.

8. Optischer Sender (31) nach Anspruch 6, wobei die Lichtaustrittsstruktur (312) eine ringförmige Struktur ist und der Testlichtfleck ein ringförmiger Lichtfleck ist.

9. Optischer Sender (31) nach Anspruch 1, wobei die Justierstruktur ein Lichtaustrittsfenster (313) umfasst, das Lichtaustrittsfenster (313) eine erste Verbindungsoberfläche (3134) umfasst, ein eingeschlossener Winkel zwischen einer Normallinie der ersten Verbindungsoberfläche (3134) und einer ersten Richtung ein erster eingeschlossener Winkel ist, der erste eingeschlossene Winkel ein spitzer Winkel ist, das optische Signal des zentralen Lichtflecks von der ersten Verbindungsoberfläche (3134) auf das Lichtaustrittsfenster (313) einfällt und die erste Richtung senkrecht zu einer originalen Richtung ist, in der das optische Signal des zentralen Lichtflecks von dem Originallichtfleck ausgesendet wird.

10. Optischer Sender (31) nach Anspruch 9, wobei das Lichtaustrittsfenster (313) ferner eine zweite Verbindungsoberfläche (3136) umfasst, die durch Verbundensein mit der ersten Verbindungsoberfläche (3134) angeordnet ist, die erste Verbindungsoberfläche (3134) nahe dem Photodetektor (33) angeordnet ist, ein eingeschlossener Winkel zwischen einer Normallinie der zweiten Verbindungsoberfläche (3136) und der ersten Richtung ein zweiter eingeschlossener Winkel ist und der zweite eingeschlossene Winkel größer als der erste eingeschlossene Winkel ist.

11. Optischer Sender (31) nach Anspruch 10, wobei das Lichtaustrittsfenster (313) ferner eine dritte Verbindungsoberfläche (3138) umfasst, die dritte Verbindungsoberfläche (3138) mit einem Ende der ersten Verbindungsoberfläche (3134), das von der zweiten Verbindungsoberfläche (3136) weg weist, verbunden ist, ein eingeschlossener Winkel zwischen einer Normallinie der dritten Verbindungsoberfläche (3138) und der ersten Richtung ein dritter eingeschlossener Winkel ist, der zweite eingeschlossene Winkel ein spitzer Winkel ist und der dritte eingeschlossene Winkel ein stumpfer Winkel ist.

12. Photoempfindliche Vorrichtung (20), umfassend den optischen Sender (31) und den Photodetektor (33) nach einem der Ansprüche 1 bis 11, wobei der Photodetektor (33) dazu konfiguriert ist, ein Rückwärtssignal zu empfangen, das erlangt wird, nachdem ein optisches Testsignal von einer Haut eines Benutzers reflektiert und/oder gestreut wurde, um ein Vitalzeichen des Benutzers zu erfassen.

13. Photoempfindliche Vorrichtung (20) nach Anspruch 12, wobei der Photodetektor (33) einen Detektorkörper (331) und ein optisches Empfangsfenster (333) umfasst, das Rückwärtssignal durch das optische Empfangsfenster in den Detektorkörper (331) eintritt, das optische Empfangsfenster (333) eine erste Oberfläche (3331) und eine zweite Oberfläche (3333) umfasst, die zweite Oberfläche (3333) zum Detektorkörper (331) hin angeordnet ist und die zweite Oberfläche (3333) eine geneigte Oberfläche umfasst, die in eine Richtung geneigt ist, in der sich eine Lichtquelle (311) befindet, um einen Einfallswinkel zu verringern, in dem ein Rückwärtssignal, das von der zweiten Oberfläche ausgesendet wird, auf den Detektorkörper (331) einfällt.

## Revendications

1. Transmetteur optique (31) utilisé pour la photopléthysmographie, comprenant une source de lumière (311) et une structure de réglage, dans lequel la source de lumière (311) est configurée pour émettre un point lumineux d'origine, pour transmettre un signal optique de test à une peau d'un utilisateur, la structure de réglage est située sur un trajet optique de sortie du signal optique de test, un signal optique de test transmis à partir d'un centre de point lumineux d'origine du point lumineux d'origine est un signal optique de point lumineux central, et la structure de réglage est configurée pour diffuser le signal optique de point lumineux central dans une direction éloignée du centre du point lumineux d'origine, pour convertir le point lumineux d'origine en point lumineux de test, dans lequel le transmetteur optique (31) comprend également une structure de sortie de lumière (312) disposée sur la source de lumière (311), dans lequel le signal optique de point lumineux central est incident sur la structure de réglage à l'aide de la structure de sortie de lumière (312), et dans lequel la structure de réglage comprend une structure de lentille (35), la structure de lentille (35) comprend une face incidente (351) et une face émergente (353), la face incidente est disposée vers la source de lumière (311), un évidement qui est disposé de manière concave vers un côté sur lequel la source de lumière (311) est située est disposé sur le côté émergent (353) de la structure de lentille (35), et le signal optique de point lumineux central est incident sur la structure de lentille (35) à partir d'une couverture de projection orthogonale de l'évidement sur le côté incident, et dans lequel le côté émergent (353) comporte également une surface sphérique faisant saillie dans une direction s'éloignant de la source de lumière (311), pour faire converger la lumière de bord transmise à partir d'une région de bord d'un point lumineux d'origine.

2. Transmetteur optique (31) selon la revendication 1, dans lequel la structure de réglage comprend une structure de réseau, configurée pour diffracter le signal optique de point lumineux central incident ; et/ou la structure de réglage comprend un film réfléchissant, configuré pour réfléchir le signal optique de point lumineux central incident.

3. Transmetteur optique (31) selon la revendication 1, dans lequel la structure de réglage comprend une structure de lentille (35), la structure de lentille (35) comprend un côté incident et un côté émergent, le côté incident est disposé vers la source de lumière (311), un évidement qui est disposé de manière concave vers un côté éloigné de la source de lumière (311) est disposé sur le côté incident de la structure de lentille, et le signal optique de point lumineux central peut être incident sur la structure de lentille (35) à partir de l'évidement et être transmis à partir du côté émergent.

4. Transmetteur optique (31) selon la revendication 1 ou 3, dans lequel le côté incident est fixé à la structure de sortie de lumière (312).

5. Transmetteur optique selon la revendication 1 ou 3, dans lequel le côté émergent comprend une surface sphérique faisant saillie dans une direction s'éloignant du transmetteur optique.

6. Transmetteur optique selon la revendication 1, dans lequel la structure de réglage comprend une structure de sortie de lumière (312) disposée sur la source de lumière (311), et la structure de sortie de lumière (312) est disposée en évitant un chemin optique de sortie d'origine du signal optique de point lumineux central.

7. Transmetteur optique (31) selon la revendication 6, dans lequel il existe au moins deux structures de sortie de lumière (312), et deux structures de sortie de lumière adjacentes sont disposées à un intervalle, de sorte que le point lumineux de test est un point lumineux à N points, dans lequel N est supérieur à 1.

8. Transmetteur optique (31) selon la revendication 6, dans lequel la structure de sortie de lumière (312) est une structure annulaire, et le point lumineux de test est un point lumineux annulaire.

9. Transmetteur optique (31) selon la revendication 1, dans lequel la structure de réglage comprend une fenêtre de sortie de lumière (313), la fenêtre de sortie de lumière (313) comprend une première surface de connexion (3134), un angle compris entre une ligne normale de la première surface de connexion (3134) et une première direction est un premier angle compris, le premier angle compris est un angle aigu, le signal optique de point lumineux central est incident à partir de la première surface de connexion (3134) à la fenêtre de sortie de lumière (313), et la première direction est perpendiculaire à une direction d'origine dans laquelle le signal optique de point lumineux central est transmis à partir du point lumineux d'origine.

10. Transmetteur optique (31) selon la revendication 9, dans lequel la fenêtre de sortie de lumière (313) comprend également une deuxième surface de connexion (3136) disposée en étant connectée à la première surface de connexion (3134), la première surface de connexion (3134) est disposée à proximité du photodétecteur (33), un angle compris entre une ligne normale de la deuxième surface de connexion (3136) et la première direction est un deuxième angle compris, et le deuxième angle compris est supérieur au premier angle compris.

11. Transmetteur optique (31) selon la revendication 10, dans lequel la fenêtre de sortie de lumière (313) comprend également une troisième surface de connexion (3138), la troisième surface de connexion (3138) est connectée à une extrémité qui est celle de la première surface de connexion (3134) et qui est éloignée de la deuxième surface de connexion (3136), un angle compris entre une ligne normale de la troisième surface de connexion (3138) et la première direction est un troisième angle compris, le deuxième angle compris est un angle aigu, et le troisième angle compris est un angle obtus.

12. Appareil photosensible (20), comprenant le transmetteur optique (31) et le photodétecteur (33) selon l'une quelconque des revendications 1 à 11, dans lequel le photodétecteur (33) est configuré pour recevoir un signal retour obtenu après qu'un signal optique de test est réfléchi et/ou diffusé par une peau d'un utilisateur, pour collecter un signe vital de l'utilisateur.

13. Appareil photosensible (20) selon la revendication 12, dans lequel le photodétecteur (33) comprend un corps de détecteur (331) et une fenêtre de réception optique (333), le signal retour entre dans le corps de détecteur (331) à travers la fenêtre de réception optique, la fenêtre de réception optique (333) comprend une première surface (3331) et une seconde surface (3333), la seconde surface (3333) est disposée vers le corps de détecteur (331), et la seconde surface (3333) comprend une surface inclinée vers une direction dans laquelle une source de lumière (311) est située, pour réduire un angle d'incidence auquel un signal retour transmis à partir de la seconde surface est incident sur le corps de détecteur (331).
